# EUROPEAN PATENT SPECIFICATION

(11) **EP 1 200 414 B1**
(45) Date of publication and mention of the grant of the patent: **20.04.2005**
(21) Application number: 00923205.9
(22) Date of filing: 10.04.2000
(51) Int. Cl.: C07D 239/34, C07D 403/12, C07D 409/12

(54) **PROCESS FOR THE PREPARATION OF SUBSTITUTED PYRIMIDINES**
VERFAHREN ZUR HERSTELLUNG VON SUBSTITUIERTEN PYRIMIDINEN
PROCEDE DE PREPARATION DE PYRIMIDINES SUBSTITUEES

(30) Priority: 15.04.1999 US 292442; 15.06.1999 US 333528
(43) Date of publication of application: 02.05.2002
(73) Proprietor: BASF AKTIENGESELLSCHAFT, 67056 Ludwigshafen (DE)
(72) Inventor: MEYER, Oliver, D-55218 Ingelheim (DE); GUTHEIL, Dieter, D-55545 Bad Kreuznach (DE)
(86) International application number: PCT/US2000/009522
(87) International publication number: WO 2000/063183

(56) References cited:
- EP-A- 0 115 325
- WO-A-90/06918
- WO-A-98/30549
- WO-A-98/40379
- WO-A-98/56789
- US-A- 4 824 949
- US-A- 4 831 138
- US-A- 5 824 624
- US-A- 5 840 892

## Description

### BACKGROUND OF THE INVENTION

The present invention relates to an improved process for the preparation of substituted pyrimidines.

Pyrimidines, which are substituted in the 4-position by a hydrocarbyloxy or hydrocarbylthio group are of great commercial interest as highly effective pesticides or pharmaceuticals. US Patent No. 3,498,984 discloses 2-phenyl-4-thiopyrimidines with interesting pharmaceutical properties. US Patent No. 5,824,624 describes herbicidal compositions comprising 2-phenyl-4-oxypyrimidines. The International Patent Applications WO 98/40379 and WO 98/56789 disclose herbicidal 4-oxypyrimidines, in which a 5-membered heteroaromatic group is attached to the 2-position of the pyrimidine moiety.

These compounds can be prepared for example in a multi-step process including the steps of treating a benzamidine hydrochloride with a substituted acetylacetate in the presence of a strong base to form a 2-phenylpyrimid-4-one, which is subsequently treated with a halogenating agent, in particular a phosphoryl halide to yield a 4-halo-2-phenylpyrimidine, which is reacted with an alcohol or a thioalcohol.

However, this process cannot be used for manufacture of relatively large quantities on an industrial scale due to the high risk of uncontrollable heat generation during the aqueous work up of the halogenation step.

W. Schroth et al., Z. Chem. 24 (1984), 435-436 disclose the preparation of 1,3-thiazin-6-thiones by condensation of 3,3-dichloroacrolein and thioamides in the presence of trifluoroborane.

However, there is no motivation to apply this reaction on the manufacture of substituted pyrimidines, especially, since trifluoroborane is not applicable in large scale productions.

### SUMMARY OF THE INVENTION

The present invention provides an effective and efficient process for the preparation of substituted pyrimidines of formula I, 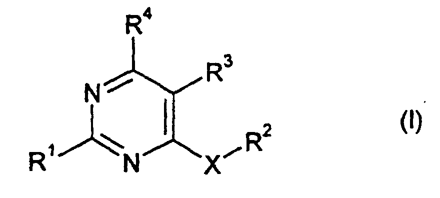 wherein
R¹ and R² each independently represent an optionally substituted alkyl, cycloalkyl, phenyl or heteroaryl group,
R³ and R⁴ each independently represent a hydrogen atom or an optionally substituted alkyl or phenyl group, and
X represents O or S,
which comprises
reacting an amidine of formula II, 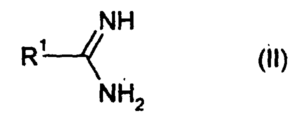 or a salt thereof, wherein R¹ has the meaning given for formula I,
with a 3,3-disubstituted vinylcarbonyl compound of formula III 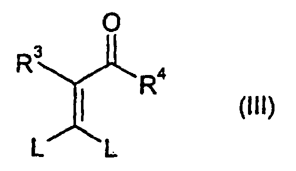 wherein R³ and R⁴ have the meaning given, and
L represent a halogen atom or a group of formula -X-R²,
(a) in an inert solvent, in the presence of a base and a compound of formula IV

   H-X-R² (IV)

   wherein X and R² have the meaning given, in the event that L represent a halogen atom, or
(b) in an inert solvent and in the presence of a base, in the event that L represents a group of formula -X-R²;
and wherein the alkyl or cycloalkyl moieties have up to 10 carbon atoms;
and
wherein the optional substituents of an optionally substituted alkyl or cycloalkyl group include phenyl, halogen atoms, nitro, cyano, hydroxyl, C₁-C₄-alkoxy, C₁-C₄-haloalkoxy and C₁-C₄-alkoxycarbonyl groups; and
wherein an heteroaryl moiety in a nitrogen containing 5- or 6-membered heteroaromatic radical; and
wherein the optional substituents of an optionally substituted phenyl or heteroaryl group include halogen, nitro, cyano, amino, hydroxyl, C₁-C₄-alkyl, C₁-C₄-alkoxy, C₁-C₄-haloalkyl, C₁-C₄-haloalkoxy, C₁-C₄-haloalkylthio and halosulfanyl.

It is, therefore, an object of the present invention to provide an efficient new process for the preparation of substituted pyrimidines. Other objects and advantages of the present invention will be apparent to those skilled in the art from the following description and the appended claims.

### DETAILED DESCRIPTION OF THE PREFERRED EMBODIMENTS

In relation to moieties defined above as comprising an optionally substituted alkyl or cycloalkyl group, specific examples of such substituents include phenyl, halogen atoms, nitro, cyano, hydroxyl, C₁₋₄-alkoxy, C₁₋₄-haloalkoxy and C₁₋₄-alkoxycarbonyl groups.

In relation to moieties defined above as comprising an optionally substituted phenyl or heteroaryl group, optional substituents include halogen, especially fluorine, chlorine and bromine atoms, and nitro, cyano, amino, hydroxyl, C₁₋₄-alkyl, C₁₋₄-alkoxy, C₁₋₄-haloalkyl, C₁₋₄-haloalkoxy, C₁₋₄-haloalkylthio and halosulfanyl groups such as SF₅. 1 to 5 substituents may suitably be employed, 1 to 2 substituents being preferred. Typically haloalkyl, haloalkoxy and haloalkylthio groups are trifluoromethyl, trifluoromethoxy, difluoromethoxy and trifluoromethylthio groups.

In general terms, unless otherwise stated herein, the term alkyl as used herein with respect to a radical or moiety refers to a straight or branched chain radical or moiety. As a rule, such radicals have up to 10, in particular up to 6 carbon atoms. Suitably an alkyl moiety has from 1 to 6 carbon atoms, preferably from 1 to 3 carbon atoms. A preferred alkyl moiety is the methyl or especially the ethyl group.

In general terms, unless otherwise stated herein, the term cycloalkyl as used herein with respect to a radical or moiety refers to a cycloalkyl radical which has up to 10, in particular up to 8 carbon atoms. Suitably a cycloalkyl moiety has from 3 to 6 carbon atoms, preferably from 3 or 6 carbon atoms. A preferred cycloalkyl moiety is the cyclopropyl, cyclopentyl and the cyclohexyl group.

In general terms, unless otherwise stated herein, the term heteroaryl as used herein with respect to a radical or moiety refers to a nitrogen containing 5- or 6-membered heteroaromatic radical or moiety.
As a rule, such radicals exhibit at least one nitrogen atom and in the case of the five-membered radicals optionally one oxygen or sulfur atom; they are preferably selected from the 5-membered azoles, diazoles, triazoles, thiazoles, isothiazoles, thiadiazoles, in particular pyrrole and pyrazole and the 6-membered azines and diazines, in particular pyridine, pyrimidine, pyridazine and pyrazine.

There may be one or more of the same or different substituents present in each part of the molecules.

In a preferred embodiment R' represents an optionally substituted phenyl, pyrid-3-yl, pyridazine-2-yl, pyrazine-3-yl, thiazol-2-yl, oxazol-2-yl, 1,3,4-thiadiazol-2-y, 1,2,4-oxadiazol-2-yl, 1,3,4-oxadiazol-2-yl, pyrazol-1-yl or C₃₋₆ cycloalkyl group.

In a preferred embodiment R² represents an optionally substituted phenyl, pyrid-2-yl, pyrid-3-yl, pyrid-4-yl, pyrazol-5-yl, pyridazine-2-yl or C₃₋₆ cycloalkyl group.

The groups R¹ and R² each independently are preferably substituted by one or more alkyl, fluoroalkyl, alkoxy or fluoroalkoxy group.

Suitable bases are weak organic or inorganic bases, preferably alkali hydrogencarbonates, such as sodium hydrogencarbonate, alkali carbonates, such as potassium carbonate or sodium carbonate, and tertiary amines, such as pyridine or triethylamine.

Further preferred embodiments of the process according to the present invention are a processes wherein:
- the reaction is carried out in the presence of a base selected from the group consisting of, alkali carbonates and tertiary amines, in particular potassium carbonate or sodium carbonate;
- the amidine of formula II to 3,3-disubstituted vinylcarbonyl compound of formula III molar ratio is from 1 : 5 to 1 : 0.5, in particular from 1 : 1.5 to 1 : 0.7, most preferred from 1 : 1.1 to 1 : 0.9;
- the reaction step further comprises stirring a mixture consisting essentially of the amidine of formula II, the 3,3-disubstituted vinylcarbonyl compound of formula III, an inert diluent, a base and a compound of formula IV (H-X-R²) which is an optionally substituted alcohol, thioalcohol, phenol or thiophenol at a temperature from 0°C to 150 °C, preferably from 60 °C to 145 °C; in particular from 80 °C to 140°C, most preferred at about the boiling point of the diluent;
- the inert diluent is selected from the group consisting of acetonitrile, benzene, toluene, xylene, hexane, cyclohexane, dichloromethane, tetrachloromethane, diethylether, diisopropylether, tertbutylmethylether, 2,2-dimethoxypropane, dimethoxyethane, diethoxyethane, tetrahydrofuran, tetrahydropyran, dimethylformamide, dimethylacetamide, N-methylpyrrolidone, dimethylsulfoxide and dioxane, and a mixture thereof, in particular toluene, dimethoxyethane or acetonitrile;
- R¹ represents a phenyl group which is substituted by at least one halogen atom, or at least one alkyl, alkoxy, haloalkyl or haloalkoxy group, in particular a phenyl group which is substituted by one or two chlorine or fluorine atoms, or one or two C₁-₄ alkyl, C₁-₄ alkoxy, C₁-₄ fluoroalkyl or C₁-₄ fluoroalkoxy groups, R¹ is most preferably a 4-trifluoromethylphenyl, difluoromethoxypyrid-2-yl or 1-methyl-3-trifluoromethylpyrazol-5-yl group;
- R¹ and R² each independently represent a phenyl group which is substituted by at least one halogen atom, and/or at least one alkyl, alkoxy, haloalkyl or haloalkoxy group, X represents O, and which comprises that the reaction step is carried out in the presence of a phenol, which is substituted by at least one halogen atom, and/or at least one alkyl, alkoxy, haloalkyl or haloalkoxy group, in particular a phenol which is substituted by one or two chlorine or fluorine atoms, or one or two C₁₋₄ alkyl, C₁₋₄ alkoxy, C₁₋₄ fluoroalkyl or C₁₋₄ fluoroalkoxy groups, most preferred a 3-trifluoromethylphenol;
- wherein the 3,3-disubstituted vinylcarbonyl compound of formula III is 3,3-dichloroacrolein.

The compounds of formula II or the salts thereof are preferably optionally substituted benzamidines or benzamidinium salts; most preferred 4-trifluoromethylbenzamidine, which can be prepared from commercially available optionally substituted benzonitriles, in particular 4-trifluoromethylbenzonitril, by addition of ammonia or ammonium salts.

Preferred benzamidinium salts are chlorides, sulfates, nitrates and carboxylates; in particular acetates and thioglycolates.

The 3,3-disubstituted vinylcarbonyl of formula III, wherein L represents a halogen atom are commercially available or can be prepared by reaction of tetrahalomethanes with vinylethers.

In a preferred embodiment of this invention the 1,1,1,3-tetrahalo-3-alkoxypropane obtained in this reaction can be hydrolysed *in-situ* to obtain the corresponding vinylcarbonyl compound of formula III, which is subsequently reacted, preferably without further isolation and/or purification steps, i.e. in a one-pot-synthesis, with the compound of formula II.

The 3,3-disubstituted vinylcarbonyl of formula III, wherein L represents a group of formula -X-R², can be prepared by reaction of a compound of formula III, wherein L represents a halogen atom with a compound of formula IV

H-X-R² (IV)

optionally in the presence of a base.

As a rule the reaction between the amidine of formula II, the 3,3-disubstituted vinyl carbonyl compound of formula III and optionally the alcohol, phenol, thioalcohol or thiophenol is carried out at elevated temperatures, preferably between 35°C and 150°C, in particular between 80°C and 145°C, most preferred at boiling point of the diluent.

The crude product obtained can be purified according to standard methods for example by distillation in vacuo, chromatographic methods or crystallization.

The reaction is as a rule completed within 5 to 50 hours, in particular 10 to 25 hours.

In a particularly preferred embodiment of the process according to this invention 3,3-dichloroacrolein (1 mole) optionally diluted with an inert diluent, in particular acetonitrile, is added to a mixture consisting of a benzamidine of formula II, wherein R¹ is a an optionally substituted phenyl group, in particular 4-trifluoromethylbenzamidine (1 mole), an optionally substituted phenol, in particular 3-trifluoromethylphenol (1.1 moles), potassium carbonate (3 to 5 moles) and a diluent, which is stirred under reflux. The reaction mixture is stirred for 10 to 40 hours under reflux and subsequently cooled down to ambient temperature, and filtered. The organic phase is concentrated in vacuo. The residue is washed with an organic solvent and the solvent is distilled off. The residue is purified by chromatography.

The compounds of formula I obtainable according to the process of this invention are partly known and partly novel.

In order to facilitate a further understanding of the invention, the following illustrative examples are presented.

### Example 1

### Preparation of 4-(3-trifluoromethylohenoxy)-2-(4-trifluoromethylphenyl)pyrimidine

3,3-Dichloroacrolein (10 mmoles) diluted with acetonitrile (50 ml), is slowly added to a mixture consisting of a 4-trifluoromethylbenzamidine (10 mmoles), 3-trifluoromethylphenol (11 mmoles), potassium carbonate (40 mmoles) and acetonitrile (100 ml), which is stirred under reflux. When the addition of 3,3-dichloroacrolein is completed additional 4-trifluoromethylbenzamidine (0.5 mmoles) is added. The reaction mixture is stirred for 20 hours under reflux and subsequently cooled down to ambient temperature and filtered through silica. The organic phase is washed with ethyl acetate and concentrated in vacuo. The residue is purified by chromatography an A1203 (petrol ethers / ethyl acetate : 2 / 1) to yield 3.25 g (85 %) of the pure product having a melting point of 66 - 67°C.

Analogously are prepared
3-methyl-4-(3-trifluoromethylphenoxy)-2-(4-trifluoromethylphenyl)-pyrimidine,
5-methyl-4-(3-trifluoromethylphenoxy)-2-(4-trifluoromethylphenyl)-pyrimidine,
4-phenoxy-2-(4-trifluoromethylphenyl)-pyrimidine

### Examples 2 to 8

### Preparation of 4-(3-trifluoromethylphenoxy)-2-(4-trifluoromethylphenyl)-pyrimidine

Analogously to example 1 4-trifluoromethylbenzamidine or the salts thereof are reacted with 3,3-dichloroacrolein in the presence of 3-trifluoromethylphenol in different solvents at different temperatures

The reactants and solvents, the reaction temperature and yields are shown in table I in which the following abbreviations have been used:

| | |
|---|---|
| TFBA | 4-trifluoromethylbenzamidine |
| TFBA * HCl | 4-trifluoromethylbenzamidine hydrochloride |
| TFBA * Ac | 4-trifluoromethylbenzamidinium acetate |
| TFBA * TG | 4-trifluoromethylbenzamidinium thioglycolate |
| TBME | *tert*-butylmethylether |
| DME | dimethoxyethane |

**Table I**

| Examples 2 to 8 | | | | |
|---|---|---|---|---|
| Example | starting material | solvent | temperature | Yield (%) |
| 2 | TFBA | acetonitril | reflux | 85 |
| 3 | TFBA | DME | reflux | 85 |
| 4 | TFBA | toluene | 90 °C | 72 |
| 5 | TFBA | TBME | reflux | 39 |
| 6 | TFBA * HCl | DME | reflux | 84 |
| 7 | TFBA * Ac | DME | reflux | 81 |
| 8 | TFBA * TG | DME | reflux | 47 |

### Example 9

### Preparation of 4-(3-trifluoromethylphenoxy)-2-(4-trifluoromethylphenyl)-pyrimidine

A mixture of 3,3-bis-(3-trifluoromethylphenoxy)-acrolein (10 mmoles), 4-trifluoromethylbenzamidine (10 mmoles), potassium carbonate (10 mmoles) and acetonitrile (100 ml), is stirred at 80 °C for four hours. The reaction mixture is cooled down to ambient temperature and filtered through silica. The organic phase is washed with ethyl acetate and concentrated in vacuo. The residue is purified by chromatography on Al₂O₃ (petrol ethers / ethyl acetate : 2 / 1) to yield 3.06 g (80 %) of the pure product having a melting point of 66 °C.

### Example 10

### Preparation of 4-(3-trifluoromethylphenoxy)-2-(4-trifluoromethylphenyl)-pyrimidine

3-Trifluoromethylphenol (5 mmoles) and subsequently 3,3-dichloroacrolein (5 mmoles) are added to a mixture consisting of a 4-trifluoromethylbenzamidinium acetate (5 mmoles), sodium carbonate (40 mmoles) and acetonitrile (35 ml), which is stirred under reflux. The reaction mixture is stirred for 20 hours under reflux and subsequently cooled down to ambient temperature and filtered through silica. The organic phase is washed with ethyl acetate and concentrated in vacuo. The residue is purified by chromatography on Al₂O₃ (petrol ethers / ethyl acetate : 2 / 1) to yield 1.1 g (60 %) of the pure product having a melting point of 66-67 °C.

### Example 11

### Enhanced preparation of 4-(3-trifluoromethylphenoxy)-2-(4-trifluoromethylphenyl)-pyrimidine

Water (20 mmoles) is added to a solution of 1,1,1,3-tetrachloro-3-ethoxypropane (10 mmoles) in dimethoxyethane (25 ml). The reaction mixture is stirred for 2 h under reflux. The resulting mixture is slowly added to a mixture consisting of a 4-trifluoromethylbenzamidine hydrochloride (10 mmoles), 3-trifluoromethylphenol (11 mmoles), potassium carbonate (60 mmoles) and dimethoxyethane (50 ml), which is stirred under reflux. When the addition of 3,3-dichloroacrolein solution is completed additional 4-trifluoromethylbenzamidine hydrochloride (1 mmoles) is added. The reaction mixture is stirred for 2 hours under reflux and subsequently cooled down to ambient temperature, filtered through silica, and the organic phase is concentrated in vacuo. The residue is purified by chromatography on SiO₂ (petrol ethers / diisopropylether : 6 / 1) to yield 3,07 g (80 %) of the product having a melting point of 66-67 °C.

### Example 12

### Preparation of 2-(4-chlorophenyl)-4-(3-trifluoromethylphenoxy)pyrimidine

3,3-Dichloroacrolein (10 mmoles) diluted with dimethoxyethane (35 ml), is slowly added to a mixture consisting of a 4-chlorobenzamidine hydrochloride (10 mmoles), 3-trifluoromethylphenol (11 mmoles), potassium carbonate (40 mmoles) and dimethoxyethane (40 ml), which is stirred under reflux. When the addition of 3,3-dichloroacrolein is completed additional 4-chlorobenzamidine hydrochloride (1 mmoles) is added. The reaction mixture is stirred for 3 hours under reflux and subsequently cooled down to ambient temperature over night and filtered through silica. The organic phase is concentrated in vacuo. The residue was purified by chromatography on Al₂O₃ (petrol ethers/ethyl acetate: 20/1) to yield 2.79 g (80 %) of the pure product having a melting point of 92 °C.

### Example 13

### Preparation of 4-(3-trifluoromethylphenoxy)-2-(4-fluorophenyl)pyrimidine

3,3-Dichloroacrolein (10 mmoles) diluted with dimethoxyethane (35 ml), is slowly added to a mixture consisting of a 4-fluorobenzamidine acetate (10 mmoles), 3-trifluoromethylphenol (11 mmoles), potassium carbonate (40 mmoles) and dimethoxyethane (40 ml), which is stirred under reflux. When the addition of 3,3-dichloroacrolein is completed additional 4-fluorobenzamidine acetate (1 mmoles) is added. The reaction mixture is stirred for 3 hours under reflux and subsequently cooled down to ambient temperature over night and filtered through silica. The organic phase is concentrated in vacuo. The residue was purified by chromatography on Al₂O₃ (petrol ethers / ethyl acetate : 20 / 1 ) to yield 2.52 g (75 %) of the pure product having a melting point of 52 °C.

### Example 14

### Preparation of 2-cyclopropyl-4-(3-trifluoromethylphenoxy)pyrimidine

3,3-Dichloroacrolein (10 mmoles) diluted with dimethoxyethane (35 ml), is slowly added to a mixture consisting of a cyclopropylcarbamidine hydrochloride (10 mmoles), 3-trifluoromethylphenol (11 mmoles), potassium carbonate (40 mmoles) and dimethoxyethane (40 ml), which is stirred under reflux. When the addition of 3,3-dichloroacrolein is completed additional cyclopropylcarbamidine hydrochloride (1 mmoles) is added. The reaction mixture is stirred for 3 hours under reflux and subsequently cooled down to ambient temperature over night and filtered through silica. The organic phase is concentrated in vacuo. The residue was purified by chromatography on Al₂O₃ (petrol ethers / ethyl acetate : 20 / 1) to yield 2.1 g (75 %) of the pure product having as a colorless liquid; ¹H NMR (CDCl₃); δ = 2.10 ppm (m, N=C(=N)-CH).

### Example 15

### Preparation of 2-(4-fluorophenyl)-4-(5-trifluoromethyl-2-methylpyrazol-3-yloxy)pyrimidine

Water (20 mmoles) is added to a solution of 1,1,1,3-tetrachloro-3-ethoxypropane (10 mmoles) in dimethoxyethane (25 ml). The reaction mixture is stirred for 61/2 h at 40°C. The resulting mixture is slowly added to a mixture consisting of a 4-fluorobenzamidine hydrochloride (10 mmoles), 4-trifluormethyl-2-methylpyrazol-1-on (11 mmoles), potassium carbonate (60 mmoles) and dimethoxyethane (50 ml), which is stirred under reflux. When the addition of 3,3-dichloroacrolein solution is completed additional 4-fluorobenzamidine hydrochloride (0.5 mmoles) is added. The reaction mixture is stirred for 2 hours under reflux and subsequently cooled down to ambient temperature, filtered through silica, and the organic phase is concentrated in vacuo. The residue is purified by chromatography on SiO₂ (petrol ethers / ethylacetate : 2 / 1) to yield 1.40 g (46 %) of beige crystals having a melting point of 101-102 °C.

### Example 16

### Preparation of 4-(2-difluoromethoxypyridin-4-yloxy)-2-(pyrazin-2-yl)-pyrimidine

Water (20 mmoles) is added to a solution of 1,1,1,3-tetrachloro-3-ethoxypropane (10 mmoles) in dimethoxyethane (25 ml). The mixture is stirred for 2 h at 60°C. The resulting mixture is slowly added to a mixture consisting of a pyrazine-2-carboxamidine hydrochloride (10 mmoles), 2-difluoromethoxypyridin-4-ol (10 mmoles), potassium carbonate (60 mmoles) and dimethoxyethane (50 ml), which is stirred under reflux. When the addition of 3,3-dichloroacrolein solution is completed additional pyrazine-2-carboxamidine hydrochloride (0.5 mmoles) is added. The reaction mixture is stirred for 2 hours under reflux and subsequently cooled down to ambient temperature, filtered through silica, and the organic phase is concentrated in vacuo. The residue is purified by chromatography on SiO₂ (ethyl acetate) to yield 2,60 g (82 %) of beige crystals having a melting point of 128-129 °C.

### Example 17

### Preparation of 4-(3-trifluoromethylphenoxy)-2-(3,5-dimethylpyrazol-1-yl)-pyrimidine

Water (20 mmoles) is added to a solution of 1,1,1,3-tetrachloro-3-ethoxypropane (10 mmoles) in dimethoxyethane (25 ml). The mixture is stirred for 2 h at 90°C. The resulting mixture is slowly added to a mixture consisting of a 3,5-dimethylpyrazole-1-carboxamidine nitrate (10 mmoles), 3-trifluoromethylphenol (10 mmoles), potassium carbonate (60 mmoles) and dimethoxyethane (50 ml), which is stirred under reflux. When the addition of 3,3-dichloroacrolein solution is completed additional 3,5-dimethylpyrazole-1-carboxamidine nitrate (0.5 mmoles) is added. The reaction mixture is stirred for 10 hours under reflux and subsequently cooled down to ambient temperature, filtered through silica, and the organic phase is concentrated in vacuo. The residue is purified by chromatography on SiO₂ (ethyl acetate) to give 2,2 g of a yellow solid. The solid was washed with petrol ether (50 ml) to yield 1,75 g (52 %) of colorless crystals having a melting point of 101-102 °C.

## Claims

1. A process for the preparation of substituted pyrimidines of formula I, 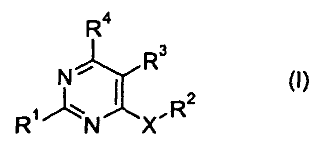 wherein
R¹ and R² each independently represent an optionally substituted alkyl, cycloalkyl, phenyl or heteroaryl group,
R³ and R⁴ each independently represent a hydrogen atom or an optionally substituted alkyl or phenyl group, and
X represents O or S,
which comprises
reacting an amidine of formula II, 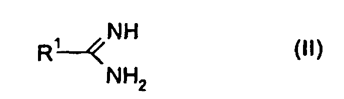 or a salt thereof, wherein R¹ has the meaning given for formula I, with a 3,3-disubstituted vinylcarbonyl compound of formula III 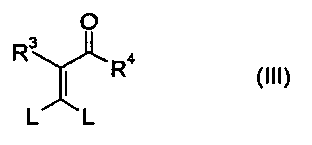 wherein R³ and R⁴ have the meaning given, and
L represent a halogen atom or a group of formula -X-R²,
(a) in an inert solvent, in the presence of a base and a compound of formula IV
H-X-R² (IV)
wherein X and R² have the meaning given, in the event that L represent a halogen atom, or
(b) in an inert solvent and in the presence of a base, in the event that L represents a group of formula -X-R²;
and
wherein the alkyl or cycloalkyl moieties have up to 10 carbon atoms;
and
wherein the optional substituents of an optionally substituted alkyl or cycloalkyl group include phenyl, halogen atoms, nitro, cyano, hydroxyl, C₁-C₄-alkoxy, C₁-C₄-haloalkoxy and C₁-C₄-alkoxycarbonyl groups; and
wherein an heteroaryl moiety is a nitrogen containing 5- or 6-membered heteroaromatic radical; and
wherein the optional substituents of an optionally substituted phenyl or heteroaryl group include halogen, nitro, cyano, amino, hydroxyl, C₁-C₄-alkyl, C₁-C₄-alkoxy, C₁-C₄-haloalkyl, C₁-C₄-haloalkoxy, C₁-C₄-haloalkylthio and halosulfanyl.

2. A process according to claim 1 which comprises that the reaction is carried out in the presence of a base selected from the group consisting of alkali hydrogencarbonates, alkali carbonates and tertiary amines.

3. A process according to claim 1, wherein the amidine of formula II to 3,3-disubstituted vinylcarbonyl compound of formula III molar ratio is from 1 : 5 to 1 : 0.5.

4. A process according to claim 1, wherein the reaction step further comprises stirring a mixture consisting essentially of the amidine of formula II, the 3,3-disubstituted vinylcarbonyl compound of formula III, the inert diluent, a base and a compound of formula IV (H-X-R²) which is an optionally substituted alcohol, thioalcohol, phenol or thiophenol at a temperature from 0°C to 150 °C.

5. A process according to claim 4, wherein the inert diluent is selected from the group consisting of acetonitrile, benzene, toluene, xylene, hexane, cyclohexane, dichloromethane, tetrachloromethane, diethylether, diisopropylether, *tert*-butylmethylether, 2,2-dimethoxypropane, dimethoxyethane, diethoxyethane, tetrahydrofuran, tetrahydropyran, dimethylformamide, dimethylacetamide, N-methylpyrrolidone, dimethylsulfoxide and dioxane, and a mixture thereof.

6. A process according to claim 1, wherein R¹ represents a phenyl group which is substituted by at least one halogen atom, or at least one alkyl, alkoxy, haloalkyl or haloalkoxy group.

7. A process according to claim 6, wherein R¹ represents a 4-trifluoromethylphenyl group.

8. A process according to claim 1 for the preparation of a 4-phenoxy-2-arylpyrimidine of formula I, wherein
R¹ and R² each independently represent a phenyl group which is substituted by at least one halogen atom, and/or at least one alkyl, alkoxy, haloalkyl or haloalkoxy group, and
X represents O,
which comprises that the reaction step is carried out in the presence of a phenol, which is substituted by at least one halogen atom, and/or at least one alkyl, alkoxy, haloalkyl or haloalkoxy group.

9. A process according to claim 8, wherein the reaction step is carried out in the presence of 3-trifluoromethylphenol.

10. A process according to claim 1, wherein the 3,3-disubstituted vinylcarbonyl compound is 3,3-dichloroacrolein.

11. A process according to claim 10, wherein 3,3-dichloroacrolein is prepared by an *in-situ* hydrolysis of a 1,1,1,3-tetrachloro-3-alkoxypropane.

## Patentansprüche

1. Verfahren zur Herstellung von substituierten Pyrimidinen der Formel I 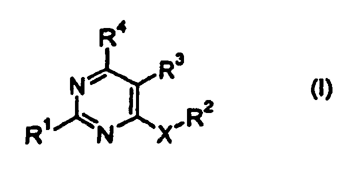 worin
R¹ und R² unabhängig voneinander jeweils für eine gegebenenfalls substituierte Alkyl-, Cycloalkyl-, Phenyl- oder Heteroarylgruppe stehen,
R³ und R⁴ unabhängig voneinander jeweils für ein Wasserstoffatom oder eine gegebenenfalls substituierte Alkyl- oder Phenylgruppe stehen und
X für O oder S steht,
bei dem man
ein Amidin der Formel II 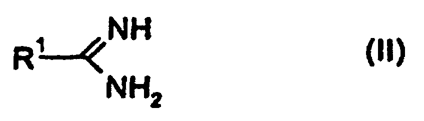 oder ein Salz davon, wobei R¹ die für Formel I angegebene Bedeutung besitzt, mit einer 3,3-disubstituierten Vinylcarbonylverbindung der Formel III 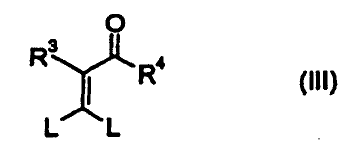 worin R³ und R⁴ die angegebenen Bedeutungen besitzen und
L für ein Halogenatom oder eine Gruppe der Formel -X-R² steht, umsetzt, und zwar
(a) für den Fall, daß L für ein Halogenatom steht, in einem inerten Lösungsmittel in Gegenwart einer Base und einer Verbindung der Formel IV
H-X-R² (IV)
worin X und R² die angegebenen Bedeutungen besitzen, oder
(b) für den Fall, daß L für eine Gruppe der Formel -X-R² steht, in einem inerten Lösungsmittel in Gegenwart einer Base; wobei die Alkyl- und Cycloalkylreste bis zu 10 Kohlenstoffatome aufweisen,
zu den fakultativen Substituenten einer gegebenenfalls substituierten Alkyl- oder Cycloalkylgruppe Phenyl, Halogenatome, Nitro-, Cyano-, Hydroxyl-, C₁-C₄-Alkoxy-, C₁-C₄-Halogenalkoxy- und C₁-C₄-Alkoxycarbonylgruppen gehören,
es sich bei einem Heteroarylrest um einen stickstoffhaltigen 5- oder 6-gliedrigen heteroaromatischen Rest handelt und
zu den fakultativen Substituenten einer gegebenenfalls substituierten Phenyl- oder Heteroarylgruppe Halogen, Nitro, Cyano, Amino, Hydroxyl, C₁-C₄-Alkyl, C₁-C₄-Alkoxy, C₁-C₄-Halogenalkyl, C₁-C₄-Halogenalkoxy, C₁-C₄-Halogenalkylthio und Halogensulfanyl gehören.

2. Verfahren nach Anspruch 1, bei dem man die Umsetzung in Gegenwart einer Base aus der Gruppe bestehend aus Alkalihydrogencarbonaten, Alkalicarbonaten und tertiären Aminen durchführt.

3. Verfahren nach Anspruch 1, bei dem das Molverhältnis von Amidin der Formel II zu 3,3-disubstituierter Vinylcarbonylverbindung der Formel III 1:5 bis 1:0,5 beträgt.

4. Verfahren nach Anspruch 1, bei dem man im Umsetzungsschritt ferner eine im wesentlichen aus dem Amidin der Formel II, der 3,3-disubstituierten Vinylcarbonylverbindung der Formel III, dem inerten Lösungsmittel, einer Base und einer Verbindung der Formel IV (H-X-R²), bei der es sich um einen gegebenenfalls substituierten Alkohol, einen gegebenenfalls substituierten Thioalkohol, ein gegebenenfalls substituiertes Phenol oder ein gegebenenfalls substituiertes Thiophenol handelt, bestehende Mischung bei einer Temperatur von 0°C bis 150°C rührt.

5. Verfahren nach Anspruch 4, bei dem man das inerte Lösungsmittel aus der Gruppe bestehend aus Acetonitril, Benzol, Toluol, Xylol, Hexan, Cyclohexan, Dichlormethan, Tetrachlormethan, Diethylether, Diisopropylether, tert.-Butylmethylether, 2,2-Dimethoxypropan, Dimethoxyethan, Diethoxyethan, Tetrahydrofuran, Tetrahydropyran, Dimethylformamid, Dimethylacetamid, N-Methylpyrrolidon, Dimethylsulfoxid und Dioxan und einem Gemisch davon auswählt.

6. Verfahren nach Anspruch 1, bei dem R¹ für eine durch mindestens ein Halogenatom oder mindestens eine Alkyl-, Alkoxy-, Halogenalkyl- oder Halogenalkoxygruppe substituierte Phenylgruppe steht.

7. Verfahren nach Anspruch 6, bei dem R¹ für eine 4-Trifluormethylphenylgruppe steht.

8. Verfahren nach Anspruch 1 zur Herstellung eines 4-Phenoxy-2-arylpyrimidins der Formel I, worin
R¹ und R² unabhängig voneinander jeweils für eine durch mindestens ein Halogenatom und/oder mindestens eine Alkyl-, Alkoxy-, Halogenalkyloder Halogenalkoxygruppe substituierte Phenylgruppe stehen und
X für O steht,
bei dem man den Umsetzungsschritt in Gegenwart eines durch mindestens ein Halogenatom und/oder mindestens eine Alkyl-, Alkoxy-, Halogenalkyl- oder Halogenalkoxygruppe substituierten Phenols durchführt.

9. Verfahren nach Anspruch 8, bei dem man den Umsetzungsschritt in Gegenwart von 3-Trifluormethylphenol durchführt.

10. Verfahren nach Anspruch 1, bei dem es sich bei der 3,3-disubstituierten Vinylcarbonylverbindung um 3,3-Dichloracrolein handelt.

11. Verfahren nach Anspruch 10, bei dem man 3,3-Dichloracrolein durch in-situ-Hydrolyse eines 1,1,1,3-Tetrachlor-3-alkoxypropans herstellt.

## Revendications

1. Procédé de préparation de pyrimidines substituées de formule I, 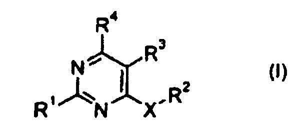 dans laquelle
R¹ et R² représentent chacun indépendamment l'un de l'autre un groupe alkyle, cycloalkyle, phényle ou hétéroaryle éventuellement substitué,
R³ et R⁴ représentent chacun indépendamment l'un de l'autre un atome d'hydrogène ou un groupe alkyle ou phényle éventuellement substitué, et
X représente O ou S,
qui comprend
la réaction d'une amidine de formule II, 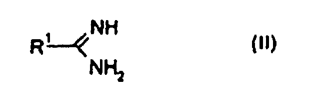 ou un sel de celle-ci, dans laquelle R¹ a la signification donnée pour la formule I, avec un composé de vinylcarbonyle 3,3-disubstitué de formule III, 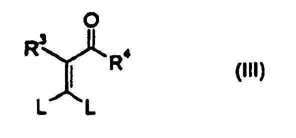 dans laquelle R³ et R⁴ ont la signification donnée, et
L représente un atome d'halogène ou un groupe de formule -X-R²,
(a) dans un solvant inerte, en présence d'une base et d'un composé de formule IV,
H-X-R² (IV)
dans laquelle X et R² ont la signification donnée, dans le cas où L représente un atome d'halogène, ou
(b) dans un solvant inerte et en présence d'une base, dans le cas où L représente un groupe de formule -X-R²;
et
dans laquelle les groupes caractéristiques alkyle ou cycloalkyle comportent jusqu'à 10 atomes de carbone;
et
dans laquelle les substituants éventuels d'un groupe alkyle ou cycloalkyle éventuellement substitué comprennent un phényle, des atomes d'halogène, des groupes nitro, cyano, hydroxyle, alcoxy en C₁-C₄, halogénoalcoxy en C₁-C₄ et alcoxycarbonyle en C₁-C₄, et
dans laquelle un groupe caractéristique hétéroaryle est un radical hétéroaromatique pentagonal ou hexagonal contenant de l'azote; et
dans laquelle les substituants éventuels d'un groupe phényle ou hétéroaryle éventuellement substitué comprennent un halogène, un nitro, un cyano, un amino, un hydroxyle, un alkyle en C₁-C₄, un alcoxy en C₁-C₄, un halogénoalkyle en C₁-C₄, un halogénoalcoxy en C₁-C₄, un halogénoalkylthio en C₁-C₄, et un halogénosulfanyle.

2. Procédé selon la revendication 1, qui comprend la réalisation de la réaction en présence d'une base choisie dans le groupe constitué d'hydrogénocarbonates alcalins, de carbonates alcalins et d'amines tertiaires.

3. Procédé selon la revendication 1, dans lequel le rapport molaire de l'amidine de formule II au composé de vinylcarbonyle 3,3-disubstitué de formule III est de 1/5 à 1/0,5.

4. Procédé selon la revendication 1, dans lequel l'étape de réaction comprend, en outre, une agitation d'un mélange constitué essentiellement de l'amidine de formule II, du composé de vinylcarbonyle 3,3-disubstitué de formule III, du solvant inerte, d'une base et d'un composé de formule IV (H-X-R²), lequel est un alcool, un thioalcool, un phénol ou un thiophénol éventuellement substitué à une température de 0°C à 150°C.

5. Procédé selon la revendication 4, dans lequel le solvant inerte est choisi dans le groupe constitué d'acétonitrile, de benzène, de toluène, de xylène, d'hexane, de cyclohexane, de dichlorométhane, de tétrachlorométhane, de diéthyléther, de diisopropyléther, de *tert*-butylméthyléther, de 2,2-diméthoxypropane, de diméthoxyéthane, de diéthoxyéthane, de tétrahydrofuranne, de tétrahydropyranne, de diméthylformamide, de diméthylacétamide, de N-méthylpyrrolidone, de diméthylsulfoxyde et de dioxanne, et un mélange de ceux-ci.

6. Procédé selon la revendication 1, dans lequel R¹ représente un groupe phényle qui est substitué par au moins un atome d'halogène ou au moins un groupe alkyle, alcoxy, halogénoalkyle ou halogénoalcoxy.

7. Procédé selon la revendication 6, dans lequel R¹ représente un groupe 4-trifluorométhylphényle.

8. Procédé selon la revendication 1, pour la préparation d'une 4-phénoxy-2-arylpyrimidine de formule I, dans laquelle
R¹ et R² représentent chacun indépendamment l'un de l'autre un groupe phényle qui est substitué par au moins un atome d'halogène, et/ou au moins un groupe alkyle, alcoxy, halogénoalkyle ou halogénoalcoxy, et
X représente O,
qui comprend la réalisation de l'étape de réaction en présence d'un phénol, lequel est substitué par au moins un atome d'halogène, et/ou au moins un groupe alkyle, alcoxy, halogénoalkyle ou halogénoalcoxy.

9. Procédé selon la revendication 8, dans lequel l'étape de réaction est réalisée en présence de 3-trifluorométhylphénol.

10. Procédé selon la revendication 1, dans lequel le composé de vinylcarbonyle 3,3-disubstitué est la 3,3-dichloroacroléine.

11. Procédé selon la revendication 10, dans lequel la 3,3-dichloroacroléine est préparée par hydrolyse *in situ* d'un 1,1,1,3-tétrachloro-3-alcoxypropane.
